(19) Europäisches Patentamt

European Patent Office

Office européen des brevets

(11) **EP 1 346 684 B1**

(12) **EUROPEAN PATENT SPECIFICATION**

(45) Date of publication and mention
of the grant of the patent:
**13.12.2006 Bulletin 2006/50**

(51) Int Cl.:
***A61B 5/00*** (2006.01)

(21) Application number: **03250663.6**

(22) Date of filing: **03.02.2003**

(54) **Apparatus and method for non-invasively measuring bio-fluid concentrations by using photoacoustic spectroscopy**

Vorrichtung und Verfahren zur nicht-invasiven Bestimmung der Konzentrationen von biologischen Flüssigkeiten mittels photoakustischer Spektroskopie

Appareil et méthode pour mesurer, de facon non invasive, des concentrations de fluides biologiques par spectroscopie photo-acoustique

(84) Designated Contracting States:
**DE FR GB**

(30) Priority: **20.03.2002 KR 2002015147**

(43) Date of publication of application:
**24.09.2003 Bulletin 2003/39**

(73) Proprietor: **SAMSUNG ELECTRONICS CO., LTD.**
**Suwon-City, Kyungki-do (KR)**

(72) Inventors:
• **Jeon, Kye-jin**
**Suwon-city,**
**Kyungki-do (KR)**

• **Hwang, In-duk**
**Suwon-city,**
**Kyungki-do (KR)**
• **Yoon, Gil-won**
**Seongdong-gu,**
**Seoul (KR)**

(74) Representative: **Ertl, Nicholas Justin**
**Elkington and Fife LLP,**
**Prospect House,**
**8 Pembroke Road**
**Sevenoaks,**
**Kent TN13 1XR (GB)**

(56) References cited:
**EP-A- 1 048 265        WO-A-02/15776**
**US-A- 4 385 634**

**Description**

The present invention relates to an apparatus and a method for non-invasively measuring bio-fluid concentrations, and more particularly, to an apparatus and a method for non-invasively measuring bio-fluid concentrations by using photoacoustic spectroscopy.

Even though research has long been carried out worldwide on a method of measuring glucose levels by means of light without collecting blood, it has never succeeded to provide any distinctive results. Various measurement techniques, such as near infrared absorption, distant infrared absorption, Ramann spectroscopy, polarization rotation, Stimulate Ramann, dispersion measurement, temperature measurement, statistical analysis, and pretreatment research, have been adopted to in vivo measure bio-fluid concentrations. However, since each of these conventional measurement techniques has its own disadvantages, it has never been easy to in vivo measure bio-fluid concentrations.

For example, near infrared absorption has the following disadvantages. First, there may not exist any absorption peak at a predetermined frequency. Second, the absorption bands of components may overlap one another. Third, it is difficult to anticipate the concentration of substance having a low concentration because dispersion easily occurs due to bio tissues. In the case of distant infrared absorption, distant infrared rays can barely penetrate the human body, even though they cause dispersion less frequently, and there exists a distinct absorption peak. In the case of Ramann spectroscopy or polarization rotation, dispersion occurs frequently due to the fact that a lot of dispersion factors exist in the human body, and thus it is difficult to precisely measure bio-fluid concentrations.

Recently, intensive research has been carried out on an apparatus and method for bio-fluid measurement by means of photoacoustic spectroscopy. When light enters a test sample, molecules are excited and collide with one another, thus generating heat. The change of heat causes the change of pressure in an airtight container, which generates an acoustic signal, e.g., a sound wave. The sound wave can be detected mostly using a microphone.

Conventional techniques using photoacoustic spectroscopy have been disclosed in European Patent No. EP0282234, PCT No. WO98/3904, European Patent No. EP0919180, and European Patent No. EP1048265. In particular, European Patent No. EP0919180 provided a method and apparatus for non-invasive measurement of blood glucose by means of photoacoustic spectroscopy, which uses a measurement cell and a reference cell as measurement devices.

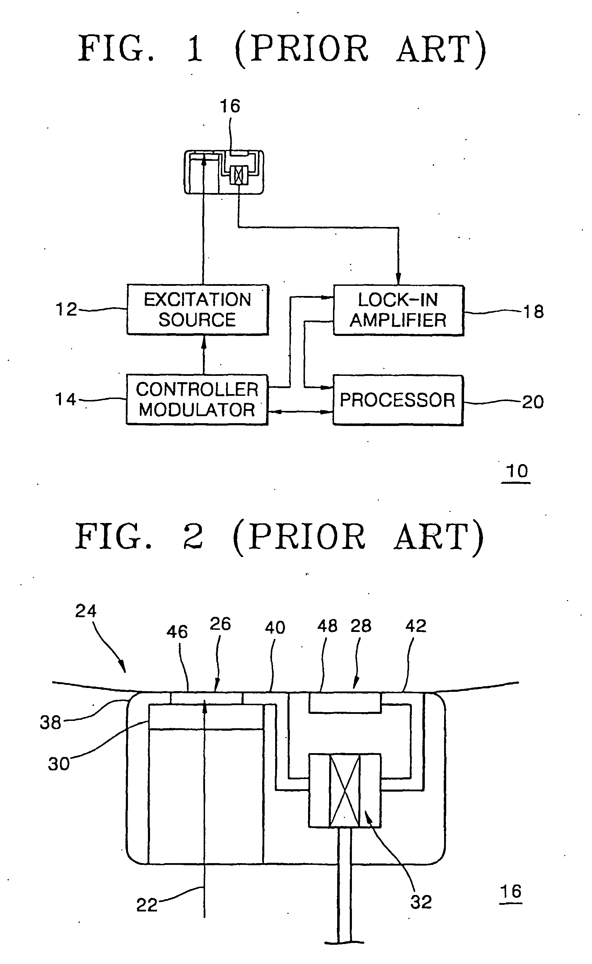

FIGS. 1 and 2 are diagrams showing a non-invasive photoacoustic measurement device disclosed in European Patent No. EP0919180. Referring to FIG. 1, a non-invasive photoacoustic measurement device 10 includes an excitation source 12, a controller/modulator 14, a probe 16, a lock-in amplifier 18, and a processor 20.

The excitation source 12 generates a sound wave when irradiated onto bio tissues, such as skin, and is transmitted to the human body through a transmitter 22, such as a bundle of optic fibers.

The probe 16, as shown in FIG. 2, includes a measurement cell 26, a reference cell 28, a window 30, and a differential microphone 32. The sound wave, generated when the excitation source 12 is irradiated onto a tissue 24, passes through the window 30 of the measurement cell 26 and heats air 38 in contact with the tissue 24 in the measurement cell 26 on a regular basis with the same modulated frequency as that of the air 38. The sound wave is absorbed into a predetermined component of the tissue 24, and the air in the measurement cell 26 keeps contracting and expanding over and over due to the periodic variation of the temperature. As a result, a periodic sound wave having the same modulated frequency as that of the air 38 is generated.

The periodic sound wave inside the measurement cell 26 is detected by the differential microphone 32, one end of which is located in the measurement cell 26 and the other end of which is located in the reference cell 28. The measurement cell 26 is located on a predetermined surface 46 of the tissue 24, onto which laser beams are irradiated, and the reference cell 28 is located on a predetermined surface 48 of the tissue 35, onto which no laser beams are irradiated.

The signals detected by the probe 16 are the outputs of the differential microphone 32 and are transmitted to the lock-in amplifier 18. Among the outputs, the lock-in amplifier 18 extracts only signals of the same frequency as the modulated frequency of the light beams that are generated and irradiated from the excitation source 12 under the control of the controller/modulator 14. The processor 20 analyzes the frequencies of the signals extracted by the lock-in amplifier 18 and derives a polarized acoustic spectrum. The conventional acoustic measurement device finds the concentration of a predetermined substance based on such a polarized acoustic spectrum.

Even though the reference cell 28 aims to compensate for noise generated by the human body, such as muscular movements, the photoacoustic measurement device disclosed in European Patent No. EP0919180 cannot precisely represent the state of the human body because it senses modulated signals only and the signals themselves have predetermined frequency bands.

The aforementioned bio-fluid measurement device using photoacoustic spectroscopy detects infrared laser beams among all laser beams irradiated on a predetermined material from a semiconductor laser, using a photoacoustic detector. Next, the bio-fluid measurement device analyzes bio-fluid concentrations based on acoustic signals detected by the photoacoustic detector. However, due to the fact that the characteristics of transmission of sound waves may vary depending on the person and the body part of the person, the conventional bio-fluid measurement device cannot precisely measure bio-fluid concentrations, which is also the problem of other conventional measurement devices using photoacoustic spectroscopy.

Document WO02/15776, which is considered to represent the closest prior art, discloses a method for assaying a component of a localized region of interest in a body comprising the steps of illuminating the region with at least one pulse of radiation having a wavelength at which the radiation is absorbed by the component to generate a change in an acoustic property of the region; transmitting ultrasound so that it is incident on the region.

According to an aspect of the present invention, there is provided an apparatus for non-invasively measuring bio-fluid concentrations. The apparatus includes a light source which irradiates on a predetermined part of a living body an optical signal having a predetermined wavelength band which can be absorbed into a predetermined component of a living body, an acoustic signal generator which generates in the vicinity of the predetermined part of the living body an acoustic signal A1 having a similar frequency band to the frequency band of a photoacoustic signal PA that is generated when the optical signal is absorbed into the predetermined component of the living body, a signal detector which detects the photoacoustic signal PA and an acoustic signal A2 that is a modulated signal of the acoustic signal A1 due to the acoustic characteristics of the living body, a controller which generates the acoustic signal A1 in a predetermined frequency band, an optical detector which detects an intensity E of the optical signal, and a calculator which calculates a signal compensation value (N) based on an intensity (E) of the optical signal from the light source, and the photoacoustic signal (PA) and the measuring at least one effect of the change on the incident ultrasound; using the measured at least one effect to determine an absorption coefficient for the radiation in the region; and using the determined absorption coefficient to determine concentration of the component in the region. acoustic signal A2 input from the detection means, and computes a concentration (C) of the predetermined component.

The invention provides an apparatus and a method for non-invasively measuring bio-fluid concentrations, the results of which can be barely affected by differences among people to be tested or among parts of the human body to be tested.

Preferably, the apparatus further includes an indicator which indicates the concentration (C) of the predetermined component.

Preferably, the signal compensation value (N) satisfies the following equation:

$$N = \frac{PA}{Es\sqrt{vA_2}} \quad \cdots (1)$$

Preferably, the concentration (C) of the predetermined component is proportional to the signal compensation value (N).

Preferably, the light detector, the controller, and the calculator are integrated into one unit, or the light detector, the controller, the calculator, and the indicator are integrated into one unit.

Preferably, the acoustic signal generator and the signal detector are integrated into one unit, or the light source, the acoustic signal generator, and the signal detector are integrated into one unit.

Preferably, the acoustic signal generator can be fixed to a human body by an air pumping method.

Preferably, the light source is any of a laser diode (LD), a light emitting diode (LED), a laser, a black body radiator, and a lamp.

According to another aspect of the present invention, there is provided an apparatus for non-invasively measuring bio-fluid concentrations. The apparatus includes a light source which irradiates on a predetermined part of a living body an optical signal having a predetermined wavelength band which can be absorbed into a predetermined component of a living body, a light detector which detects an intensity (E) of the optical signal and a photoacoustic signal generated when the optical signal is absorbed into the predetermined component of the living body, an acoustic signal generation/measurement device which generates in the vicinity of the predetermined part of the living body an acoustic signal A1 having a frequency band to the frequency band of the photoacoustic signal PA and measures an acoustic signal A2 which is a modulated signal of the acoustic signal A1 due to the acoustic characteristics of the living body, a controller which controls the acoustic signal generation/measurement device so that the acoustic signal A1 in a predetermined frequency band can be generated, and a calculator which calculates a signal compensation value (N) based on the intensity (E) of the optical signal from the light source, and the photoacoustic signal (PA) and the acoustic signal A2 input from the signal detector, and computes the concentration (C) of the predetermined component.

Preferably, the apparatus further includes an indicator which indicates the concentration (C) of the predetermined component.

Preferably, the signal compensation value (N) satisfies Equation (1) above.

Preferably, the concentration (C) of the predetermined component is proportional to the signal compensation value (N).

Preferably, the controller and the calculator are integrated into one unit, or the controller, the calculator, and the indicator are integrated into one unit.

Preferably, the acoustic signal generation/measurement device and the light detector are integrated into one unit, or the light source, the acoustic signal generation/measurement device and the light detector are integrated into one unit.

Preferably, the acoustic signal generator can be fixed to a human body by an air pumping method.

Preferably, the light source is any of a laser diode (LD), a light emitting diode (LED), a laser, a black body radiator, and a lamp.

According to still another aspect of the present invention, there is provided a method for non-invasively measuring bio-fluid concentrations. The method includes applying an optical signal having a predetermined wavelength band which can be absorbed into a predetermined component of a living body to a predetermined part of the living body, detecting the intensity (E) of the optical signal and a photoacoustic signal (PA) generated when predetermined wavelengths of the optical signal are absorbed into the predetermined component of the living body, generating an acoustic signal A1 having a similar frequency band to the frequency band of the photoacoustic signal (PA) in the vicinity of the predetermined part of the living body, detecting an acoustic signal A2 which is a modulated signal of the acoustic signal A1 due to the acoustic characteristics of the living body, and calculating a signal compensation value (N) based on the intensity of the optical signal, the photoacoustic signal PA and the acoustic signal A2, and computing a concentration (C) of the predetermined component of the living body.

Preferably, the signal compensation value (N) satisfies Equation (1).

Preferably, the concentration (C) of the predetermined component of the living body is proportional to the signal compensation value (N).

According to the present invention, it is possible to compensate for a deviation in the speeds of photoacoustic signals which are affected by differences among parts of a human body to be tested and among people to be tested by correcting the photoacoustic signal using a reference photoacoustic signal, and it is possible to preciously measure bio-fluid concentrations by correcting a variation in the transmission characteristics of photoacoustic signals, such as reflection or dispersion, which is caused by the structure of a living body.

The above and other aspects and advantages of the present invention will become more apparent by describing in detail preferred embodiments thereof with reference to the attached drawings in which:

FIG. 1 is a block diagram of a device for non-invasively measuring blood glucose using photoacoustic spectroscopy, which is disclosed in Japanese Patent Publication No. hei 11-235331;

FIG. 2 is a diagram of a device for non-invasively measuring blood glucose using photoacoustic spectroscopy, which is disclosed in Japanese Patent Publication No. hei 11-235331;

FIG. 3 is a block diagram of a device for non-invasively measuring bio-fluid concentrations according to an embodiment of the present invention;

FIG. 4 is a perspective view of a penetration-type device for non-invasively measuring bio-fluid concentrations according to an embodiment of the present invention;

FIG. 5 is a block diagram of a reflection-type device for non-invasively measuring bio-fluid concentrations according to an embodiment of the present invention;

FIG. 6 is a flowchart of a method of non-invasively measuring bio-fluid concentrations according to an embodiment of the present invention;

FIG. 7A is a graph showing the absorption spectra of glucose solutions in a range of near infrared rays;

FIG. 7B is a graph showing the absorption spectra of glucose solutions of FIG. 7A on a logarithmic scale;

FIG. 7C is an enlarged view of region B of FIG. 7A;

FIG. 7D is an enlarged view of region C of FIG. 7A; and

FIG. 8 is a graph showing the absorption spectra of glucose solutions in a range of distant infrared rays.

Hereinafter, an apparatus and a method for non-invasively measuring bio-fluid concentrations and a method thereof will be described in greater detail with reference to the accompanying drawings. The same reference numerals in different drawings represent the same elements.

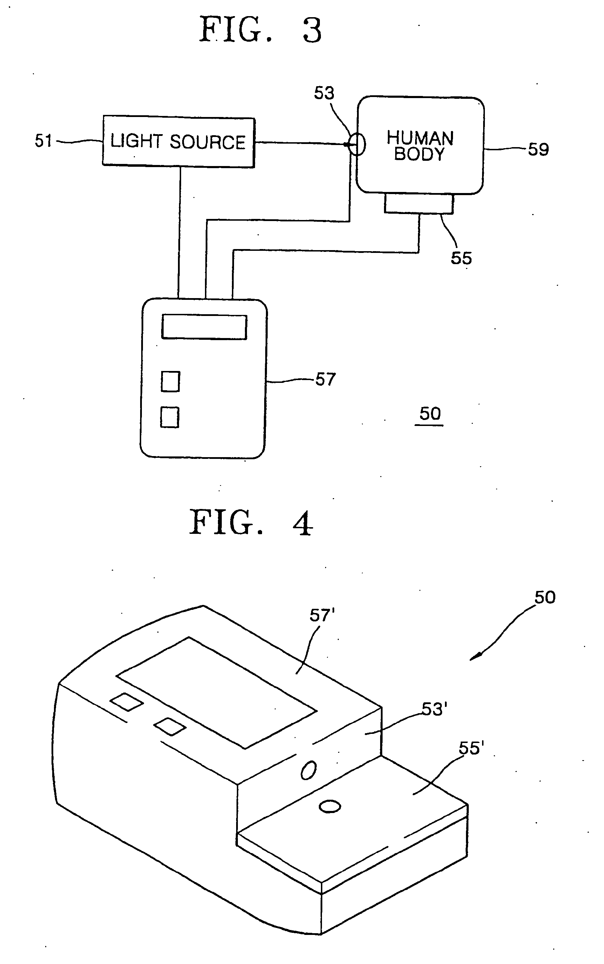

FIG. 3 is a block diagram of an apparatus for non-invasively measuring bio-fluid concentrations according to an embodiment of the present invention. Referring to FIG. 3, an apparatus 50 for non-invasively measuring bio-fluid concentrations includes a light source 51, an acoustic signal generator 53 located close to an object of measurement, to which an optical signal is applied, a signal detector 55 located close to another side of the object of measurement, an optical detector (not shown) for detecting an intensity (E) of the optical signal, a controller, a calculator, and an indicator 57. Here, the controller, the calculator, and the indicator 57 are connected to the light source 51 and the signal detector 55.

The light source 51 applies an optical signal of a predetermined frequency, which can be absorbed into a predetermined component of the human body, to a predetermined part of the human body 59.

When the optical signal penetrates the predetermined part of the human body 59, predetermined waves of a predetermined wave length band are absorbed by the predetermined component. The acoustic signal generator 53, then, detects a photoacoustic signal PA generated and modulated by the absorption of the wavelengths, using the signal detector 55. Next, the acoustic signal generator 53 generates an acoustic signal A1 having a similar frequency to that of the photoacoustic signal PA in the vicinity of the human body 59.

The signal detector 55 detects the photoacoustic signal PA which has passed through the predetermined part of the human body 59. As described above, the signal detector 55 detects an acoustic signal A2 which is generated when the acoustic signal A1 generated from the acoustic signal generator 53 passes through the predetermined part of the human body 59 and thus is modulated due to the acoustic characteristics of the human body 59.

Here, the predetermined component of the human body 59 may represent bio-fluids, such as glucose, hemoglobin, albumin, and cholesterol, which absorb light beams of predetermined wavelengths depending on their characteristics. If electrons in a bio-fluid absorb light beams, they move to a higher energy level. As time goes by, the electrons return to a lower energy level, thus generating sound waves.

The light detector measures the intensity (E) of light beams, i.e., an optical signal generated from the light source 51.

The controller controls the light source 51 and the acoustic signal generator 53 so that the acoustic signal A1 of a predetermined frequency band can be generated. The calculator calculates a signal compensation value (N) based on the intensity (E) of an optical signal input from the light source 51, and the photoacoustic signal PA and the acoustic signal A2 input from the signal detector 55. Next, the calculator calculates a concentration (C) of the predetermined component. Here, the signal compensation value (N) is proportional to the concentration (C) of the predetermined component.

The apparatus 50 for measuring bio-fluid concentrations may further include the indicator 57 which indicates the concentration (C) of the predetermined component. As shown in FIG. 3, the light detector, the controller, the calculator, and the indicator 57 are integrated into one unit in the apparatus 50 for measuring bio-fluid concentrations. The light detector, the controller, the calculator, and the indicator 57 may be integrated into one unit. Alternatively, some of the light detector, the controller, the calculator, and the indicator 57 may be integrated into one unit. For example, the controller and the calculator 57 may be integrated into one unit, and the acoustic signal generator 53 and the signal detector 55 may be integrated into another unit. Alternatively, the light source 51, the acoustic signal generator 53, and the signal detector 55 may be integrated into one unit.

FIG. 4 is a diagram of an example of the apparatus 50 of measuring bio-fluid concentrations, in which the light source 51, the acoustic signal generator 53, the signal detector 55, the light detector, the controller, the calculator, and the indicator 57 are integrated into one unit. Reference numeral 53' refers to an exit, through which an optical signal generated from the light source 51 and the acoustic signal A1 generated from the acoustic signal generator 53 are released, and reference numeral 55' refers to an entrance through which the photoacoustic signal PA2 and the acoustic signal A2 pass to enter the signal detector 55. Reference numeral 57' refers to a predetermined unit, into which a light detector, a controller, a calculator, and an indicator are integrated.

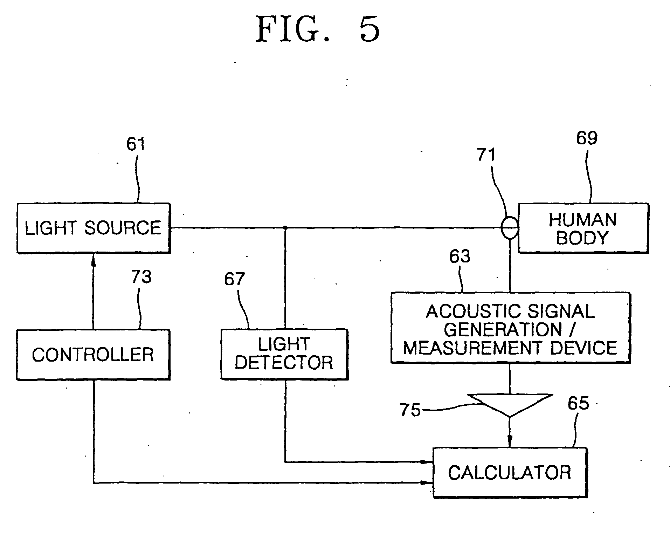

FIG. 5 is a block diagram of an apparatus for measuring bio-fluid concentrations according to another embodiment of the present invention. Referring to FIG. 5, an apparatus for measuring bio-fluid concentrations includes a light source 61, which applies an optical signal of a predetermined frequency band that can be absorbed into a part of a human body 69, and an acoustic signal generation/measurement device 63, which generates an acoustic signal A1 having a similar frequency band to that of a photoacoustic signal PA in the vicinity of the part of the human body 69. Here, the photoacoustic signal PA is generated when the optical signal is reflected by the part of the human body 69 and waves of predetermined lengths are absorbed into a predetermined component of the human body 69.

In addition, the apparatus for measuring bio-fluid concentrations further includes a light detector 67, which detects an intensity (E) of the optical signal and the photoacoustic signal PA, a controller 73, which controls the acoustic signal generation/measurement device 63 so that an acoustic signal A1 in a predetermined frequency band can be generated, and a calculator 65, which calculates a signal compensation value N based on the photoacoustic signal PA input from the light detector 67 and the acoustic signal A2 input from the acoustic signal generation/measurement device 63, and then computes a concentration (C) of the predetermined component based on the signal compensation value (N).

The apparatus for measuring bio-fluid concentrations may further include an indicator (not shown), which indicates the concentration (C) of the predetermined component. All or some of the light source 61, the controller 73, the calculator 65, the light detector 67, the acoustic signal generation/measurement device 63, and the indicator may be integrated into one unit.

Pulse-type heat expansion caused by light pulses generates acoustic pressure waves. The pressure waves (p) can be expressed by the following wave equation:

$$[\frac{1}{v^2}\frac{B^2}{Bt^2} - y^2]p = \frac{\alpha\beta}{C_p}\cdot\frac{BI}{Bt} \quad \cdots(2)$$

Here, I, $\alpha$, and $\beta$ represent the intensity of light, the coefficient of heat expansion, and the velocity of sound waves, respectively. In addition, $C_p$ and t represent specific heat and time, respectively. The amplitude (P) of Lai and Young pulse-shape photoacoustic signal can be expressed by the following formula:

$$P \propto \alpha\beta\frac{\sqrt{v}}{C_p}E \quad \cdots(3)$$

Here, E refers to the intensity of light beams incident on the predetermined part of the human body 69.

As shown in Formula (3), the photoacoustic signal can be derived in consideration of the optical characteristics of a medium, such as the intensity of incident beams or the coefficient of light absorption, the thermal characteristics of the medium, such as the coefficient of heat expansion, and the acoustic characteristics of the medium, such as the velocity of sound waves and the function of sound wave transmission. The thermal characteristics of the human body vary less considerably than the optical factors and the acoustic characteristics. Accordingly, it is possible to more precisely measure the absorption coefficient of the medium to be tested by compensating for the optical and acoustic characteristics of the human body.

In the apparatus for measuring bio-fluid concentrations and the method thereof according to the present invention, the signal compensation value (N) is derived following Equation (1) in order to compensate for the acoustic characteristics of the medium to be tested.

The signal compensation value (N) is proportional to the concentration (C) of the predetermined component, as shown in the following equations. The signal compensation value (N) can be compensated for by measuring sound waves and deriving the velocity (v) of the sound waves and the coefficient $A_2$ of sound wave transmission.

$$N = \frac{\alpha\beta \frac{\sqrt{v}}{C_p} A_2}{Es\sqrt{vA_2}} \quad \cdots (4)$$

$$\alpha = k_h N \quad \cdots (5)$$

Here, $K_h$ is equal to $\dfrac{C_p}{\beta}$. Since the coefficient of absorption can be derived from Equation (5), the concentration (C) of the target component can be calculated by comparing the coefficient of absorption of a detected signal wave with the coefficient of absorption of a reference wave in order to measure bio-fluid concentrations.

In order to calculate the signal compensation value (N), frequency analysis based on the Fourier transformation, or wavelet analysis can be performed. Alternatively, the spatial characteristics of the human body can be compensated for by using a plurality of detectors.

Since the state of tissues may vary depending on the person, the part of the human body, and the time when the tissues are tested, such variation must be compensated for. In particular, in order to get rid of the influence of other components in the human body, the concentrations of other components can be searched in advance and can be compensated for, so that the concentration of the target component, such as glucose, can be more precisely calculated. For example, the concentration of water or hemoglobin can be determined by using an optical method or by adding acoustic waves which are irradiated on tissues.

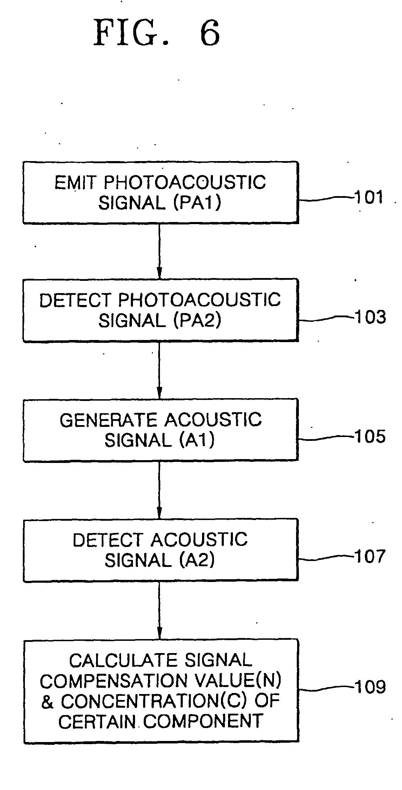

FIG. 6 is a flowchart of a method for non-invasively measuring bio-fluid concentrations using photoacoustic spectroscopy according to an embodiment of the present invention. Referring to FIG. 6, an optical signal PA1 in a predetermined wavelength band, which is emitted from a light source, is irradiated on a predetermined part of a human body to be tested in step 101. When the optical signal PA1 passes through or is reflected by the predetermined part, predetermined wavelengths are absorbed into a predetermined component of the human body, and thus a photoacoustic signal PA is generated. Then, the intensity (E) of the photoacoustic signal PA is detected in step 103. An acoustic signal A1 having a similar frequency band to that of the detected photoacoustic signal PA is generated in step 105.

When the acoustic signal A1 passes through or is reflected by the component of the human body, predetermined wavelengths are absorbed into the component of the human body, and thus an acoustic signal A2 is generated. Then, the acoustic signal A2 is detected in step 107. Next, a signal compensation value (N) or the concentration (C) of the predetermined component of the human body is calculated based on the intensity (E) of the optical signal, the detected photoacoustic signal PA, and the acoustic signal A2 in step 109.

The concentration (C) of the predetermined component can be derived based on the signal compensation value (N), following Equations (1), (4), and (5). As described above, the photoacoustic signal PA needs to be compensated for depending on the state of the human body, and its compensation can be performed using the signal compensation value (N) derived from Equation (1).

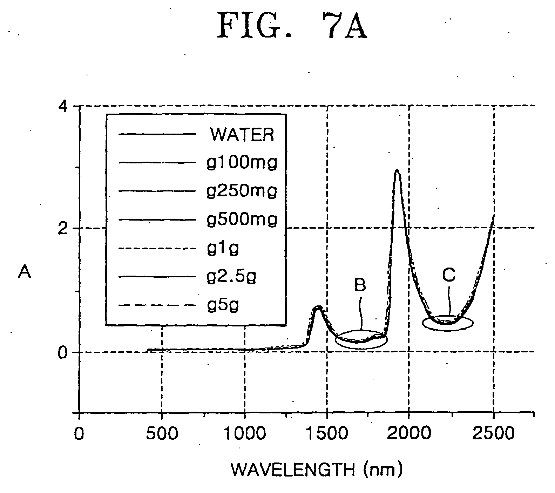

FIGS. 7A through 7D are graphs showing the absorption spectra of glucose solutions in a near infrared ray range.

FIG. 7A shows the absorption spectra of glucose solutions having different amounts of glucose, i.e., 100 mg, 250 mg, 500 mg, 1 g, 2.5 g, and 5 g, when applying a photoacoustic signal having a wavelength band ranging from 400 nm to 2500 nm to the glucose solutions. As shown in FIG. 7A, the seven glucose solutions show almost the same absorption spectrum, which means that the absorption of a photoacoustic signal varies depending on the amount of water in a glucose solution having a fine amount of glucose.

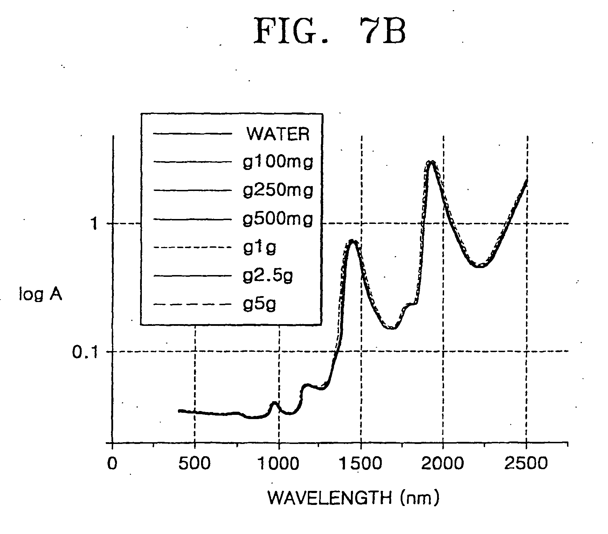

FIG. 7B is a graph showing the absorption spectra of glucose solutions of FIG. 7A on a logarithmic scale. In FIG. 7B, unlike in FIG. 7A, a lot of small peaks appear around a wavelength of 1000 nm.

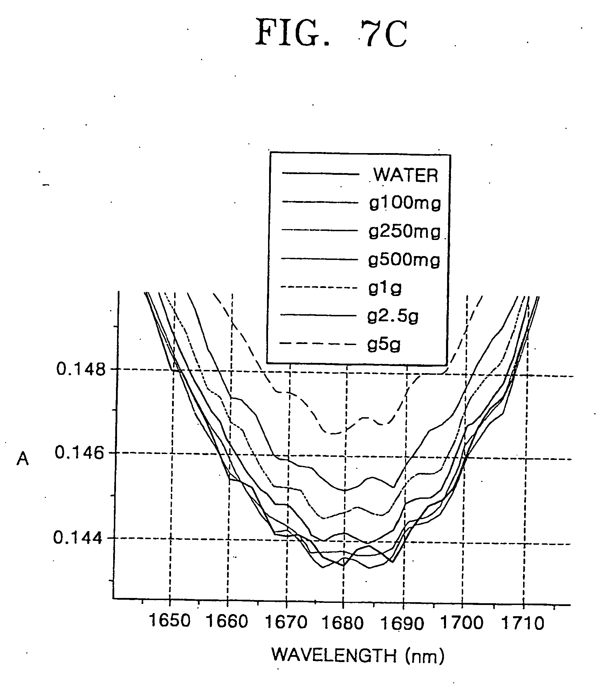

FIG. 7C is an enlarged view of region B of FIG. 7A. As shown in FIG. 7C, a glucose solution having 100 mg of glucose shows a similar absorption spectrum to that of water. On the other hand, as the amount of glucose in a glucose solution increases more than 5 g, the distance between the absorption spectrum of the glucose solution and the absorption spectrum of water becomes greater. FIG. 7C shows that the absorption spectrum of a glucose solution varies depending on the concentration when applying a photoacoustic signal having a wavelength band of 1660 nm - 1700 nm to the glucose solution.

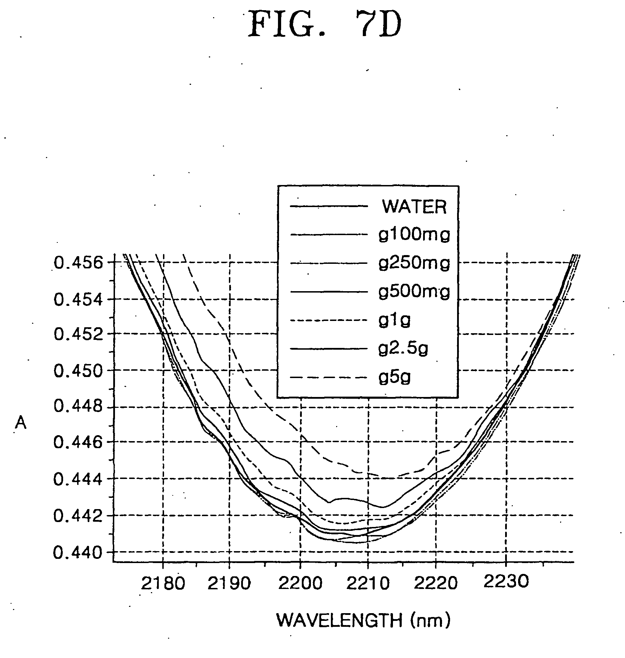

FIG. 7D is an enlarged view of region C of FIG. 7A. In FIG. 7D, the phenomenon that the more glucose in a glucose solution, the higher absorption rate of a photoacoustic signal is distinctively shown around a wavelength range of about 2190 nm - 2220 nm.

According to the results of the aforementioned experiments, it is possible to conclude that photoacoustic signals having a wavelength band of about 1660 nm - 1700 nm or a wavelength band of about 2190 - 2220 nm can be used in a near infrared ray range to measure the concentration of glucose using the apparatus for measuring bio-fluid concentrations according to an embodiment of the present invention.

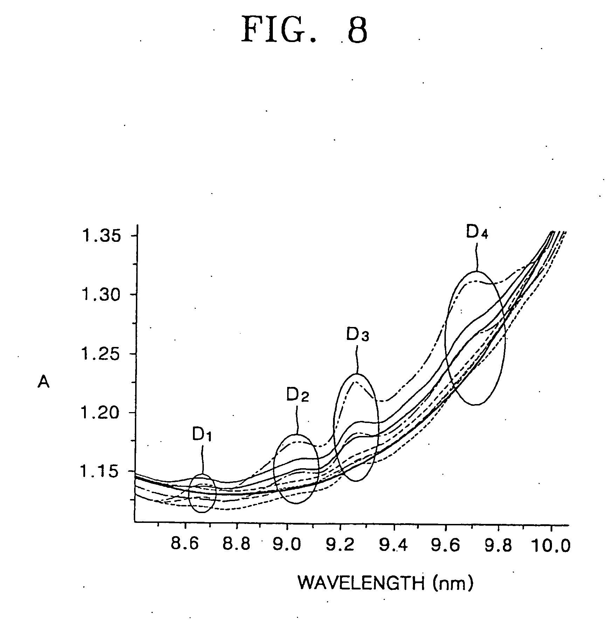

FIG. 8 is a graph showing the absorption spectra of glucose solutions in a distant infrared ray range. In FIG. 8, $D_1$, $D_2$, $D_3$, and $D_4$, at which peaks of the absorption spectra appear, represent important wavelengths bands around 8.7 nm, 9.0 nm, 9.3 nm, and 9.8 nm, respectively. In other words, when using the apparatus for measuring bio-fluid concentrations according to an embodiment of the present invention, it is preferable to use a photoacoustic signal having a wavelength range represented by $D_1$, $D_2$, $D_3$, or $D_4$ in a distant infrared ray range.

The method and apparatus for non-invasively measuring bio-fluid concentrations using photoacoustic spectroscopy according to the embodiments of the present invention can make up for the different transmission characteristics of a photoacoustic signal, which vary depending on the person and the part of the human body, by compensating for the photoacoustic signal varying depending on the kind or state of a living body.

While this invention has been particularly shown and described with reference to preferred embodiments thereof, it will be understood by those skilled in the art that various changes in form and details may be made therein without departing from the scope of the invention as defined by the appended claims. For example, it is obvious to one skilled in the art that the different transmission characteristics of a photoacoustic signal can be compensated for using other compensating values.

**Claims**

1. An apparatus for non-invasively measuring bio-fluid concentrations comprising:

a light source (61) which irradiates on a predetermined part of a living body (69) an optical signal having a predetermined wavelength band which can be absorbed into a predetermined component of a living body;
an acoustic signal generator (63) which generates in the vicinity of the predetermined part of the living body an acoustic signal A1 having a similar frequency band to the frequency band of a photoacoustic signal PA that is generated when the optical signal is absorbed into the predetermined component of the living body;
detection means which detects the photoacoustic signal PA and an acoustic signal A2 that is a modulated signal of the acoustic signal A1 due to the acoustic characteristics of the living body;
a controller (73) for controlling generation of the acoustic signal A1 in a predetermined frequency band;
an optical detector (67) which detects an intensity E of the optical signal; and
a calculator (65) which calculates a signal compensation value (N) based on an intensity (E) of the optical signal from the light source, and the photoacoustic signal (PA) and the acoustic signal A2 input from the detection means, and computes a concentration (C) of the predetermined component.

2. An apparatus as claimed in claim 1 wherein the detection means comprise signal detectors.

3. An apparatus as claimed in claim 1 wherein the detection means comprises first detection means which detects the photoacoustic signal PA and second detection means which detects the acoustic signal A2, and wherein the acoustic signal generator comprises an acoustic signal generation/measurement device which includes the second detection means, and the optical detector includes the first detection means.

4. An apparatus as claimed in any preceding claim, wherein the controller controls the acoustic signal generator so that the acoustic signal A1 in a predetermined frequency band can be generated.

5. The apparatus of any preceding claim further comprising an indicator which indicates the concentration (C) of the predetermined component.

6. The apparatus of any preceding claim, wherein the signal compensation value (N) satisfies the following equation:

$$N = \frac{PA}{Es\sqrt{vA_2}} \; .$$

7.  The apparatus of any preceding claim, wherein the concentration (C) of the predetermined component is proportional to the signal compensation value (N).

8.  The apparatus of any preceding claim, wherein the optical detector, the controller, and the calculator are integrated into one unit.

9.  The apparatus of any of any preceding claim, wherein the acoustic signal generator and detection means are integrated into one unit.

10. The apparatus of any preceding claim, wherein the controller and the calculator are integrated into one unit.

11. The apparatus of claim 5, wherein the controller, the calculator and the indicator are integrated into one unit.

12. The apparatus of claim 3, wherein the acoustic signal generator /measurement device and the optical detector are integrated into one unit.

13. The apparatus of any preceding claim, wherein the acoustic signal generator can be fixed to a human body by an air pumping method.

14. The apparatus of any preceding claim, wherein the light source is any of a laser diode (LD), a light emitting diode (LED), a laser, a black body radiator, and a lamp.

15. A method for non-invasively measuring bio-fluid concentrations comprising:

    applying an optical signal having a predetermined wavelength band which can be absorbed into a predetermined component of a living body to a predetermined part of the living body; detecting the intensity (E) of the optical signal and a photoacoustic signal (PA) generated when predetermined wavelengths of the optical signal are absorbed into the predetermined component of the living body; generating an acoustic signal A1 having a similar frequency band to the frequency band of the photoacoustic signal (PA) in the vicinity of the predetermined part of the living body; detecting an acoustic signal A2 which is a modulated signal of the acoustic signal A1 due to the acoustic characteristics of the living body; and calculating a signal compensation value (N) based on the intensity of the optical signal, the photoacoustic signal PA and the acoustic signal A2, and computing a concentration (C) of the predetermined component of the living body.

16. The method of claim 15, wherein the signal compensation value (N) satisfies the following equation:

$$N = \frac{PA}{Es\sqrt{vA_2}} \; .$$

17. The method of claim 15 or 16, wherein the concentration (C) of the predetermined component of the living body is proportional to the signal compensation value (N).

**Patentansprüche**

1.  Vorrichtung zum nicht-invasiven Messen von Biofluidkonzentrationen umfassend:

    eine Lichtquelle (61), die auf einen bestimmten Teil eines lebenden Körpers (65) ein optisches Signal einstrahlt, das ein bestimmtes Wellenlängenband aufweist, das in einer bestimmten Komponente eines lebenden Körpers absorbiert werden kann; einen Generator (63) für ein akustisches Signal, der im Bereich des bestimmten Teils des lebenden Körpers ein akustisches Signal A1 erzeugt, das ein ähnliches Frequenzband aufweist wie das Frequenzband eines photoakustischen Signals PA, das erzeugt wird, wenn das optische Signal in der bestimmten Komponente des lebenden Körpers absorbiert wird; Erfassungsmittel, das das photoakustische Signal PA und ein akustisches Signal A2, das ein moduliertes Signal des akustischen Signals A1 ist, aufgrund der akustischen Eigenschaften des lebenden Körpers erfasst; eine Steuerung (73) zum Steuern der Erzeugung des akustischen Signals A1 in einem bestimmten Frequenzband; einen optischen Detektor (67), der eine Intensität E des optischen Signals erfasst; und eine Berechnungseinheit (65), die einen Signalkompensationswert (N) ausgehend von einer Intensität (E) des optischen Signals von der Lichtquelle und dem photoakustischen Signal (PA) und dem akustischen Signal A2, das vom Erfassungsmittel eingegeben ist, errechnet und eine Konzentration (C) der bestimmten Komponente berechnet.

2.  Vorrichtung nach Anspruch 1, worin die Erfassungsmittel Signaldetektoren umfassen.

3.  Vorrichtung nach Anspruch 1, worin das Erfassungsmittel erste Erfassungsmittel umfasst, die das photoakustische Signal PA erfassen und zweite Erfassungsmittel, die das akustische Signal A2 erfassen, und worin der akustische Signalgenerator eine akustische Signalerzeugungs-/Messvorrichtung umfasst, die das zweite Erfassungsmittel beinhaltet und der optische Detektor das erste Erfassungsmittel umfasst.

4.  Vorrichtung nach einem der vorhergehenden Ansprüche, worin die Steuerung den akustischen Signalgenerator so steuert, dass das akustische Signal A1 in einem bestimmten Frequenzband erzeugt werden kann.

5.  Vorrichtung nach einem der vorhergehenden Ansprüche, ferner umfassend eine Anzeigevorrichtung, die die Konzentration (C) der bestimmten Komponente anzeigt.

6.  Vorrichtung nach einem der vorhergehenden Ansprüche, worin der Signalkompensationswert (N) die folgende Gleichung erfüllt:

$$N = \frac{PA}{Es\sqrt{vA_2}}.$$

7.  Vorrichtung nach einem der vorhergehenden Ansprüche, worin die Konzentration (C) der bestimmten Komponente zum Signalkompensationswert (N) proportional ist.

8.  Vorrichtung nach einem der vorhergehenden Ansprüche, worin der optische Detektor, die Steuerung und die Berechnungseinheit in einer Einheit integriert sind.

9.  Vorrichtung nach einem der vorhergehenden Ansprüche, worin der akustische Signalgenerator und die Erfassungsmittel in einer Einheit integriert sind.

10. Vorrichtung nach einem der vorhergehenden Ansprüche, worin die Steuerung und die Berechnungseinheit in einer Einheit integriert sind.

11. Vorrichtung nach Anspruch 5, worin die Steuerung, die Berechnungseinheit und die Anzeigeeinrichtung in einer Einheit integriert sind.

12. Vorrichtung nach Anspruch 3, worin die akustische Signalgenerator/Messvorrichtung und der optische Detektor in einer Einheit integriert sind.

13. Vorrichtung nach einem der vorhergehenden Ansprüche, worin der akustische Signalgenerator nach einem Luftpumpverfahren am menschlichen Körper befestigt werden kann.

14. Vorrichtung nach einem der vorhergehenden Ansprüche, worin die Lichtquelle eines von einer Laserdiode (LD), einer Licht emittierenden Diode (LED), einem Laser, einem schwarzen Strahler und einer Lampe ist.

15. Verfahren zum nicht-invasiven Messen von Biofluidkonzentrationen umfassend:

    Aufgeben eines optischen Signals mit einem bestimmten Wellenlängenband, das in einer bestimmten Komponente eines lebenden Körpers in einem bestimmten Teil des lebenden Körpers absorbiert werden kann;
    Erfassen der Intensität (E) des optischen Signals und eines photoakustischen Signals (PA), das erzeugt wird, wenn bestimmte Wellenlängen des optischen Signals in der bestimmten Komponente des lebenden Körpers absorbiert werden;
    Erzeugen eines akustischen Signals A1 mit einem ähnlichen Frequenzband wie das Frequenzband des photoakustischen Signals (PA) im Bereich des bestimmten Teils des lebenden Körpers;
    Erfassen eines akustischen Signals A2, das ein moduliertes Signal des akustischen Signals A1 ist, aufgrund der akustischen Eigenschaften des lebenden Körpers; und
    Errechnen eines Signalkompensationswerts (N) ausgehend von der Intensität des optischen Signals, des photoakustischen Signals PA und des akustischen Signals A2 und Berechnen einer Konzentration (C) der bestimmten Komponente im lebenden Körper.

16. Verfahren nach Anspruch 15, worin der Signalkompensationswert (N) die folgende Gleichung erfüllt:

$$N = \frac{PA}{Es\sqrt{vA_2}}.$$

17. Verfahren nach Anspruch 15 oder 16, worin die Konzentration (C) der bestimmten Komponente des lebenden Körpers zum Signalkompensationswert (N) proportional ist.

**Revendications**

1.  Appareil destiné à mesurer de manière non effractive des concentrations en fluide biologique comprenant :

• une source de lumière (61) qui irradie sur une partie prédéterminée d'un corps vivant (69) un signal optique ayant une bande de longueur d'onde prédéterminée qui peut être absorbée dans un composant prédéterminé d'un corps vivant ;
• un générateur de signal acoustique (63) qui génère à proximité de la partie prédéterminée du corps vivant un signal acoustique A1 ayant une bande de fréquence similaire à la bande de fréquence d'un signal photoacoustique PA qui est généré lorsque le signal optique est absorbé dans le composant prédéterminé du corps vivant ;
• des moyens de détection qui détecte le signal photoacoustique PA et un signal acoustique A2 qui est un signal modulé du signal acoustique A1 en raison des caractéristiques acoustiques du corps vivant ;
• un dispositif de commande (73) destiné à commander la génération du signal acoustique A1 dans une bande de fréquence prédéterminée ;
• un détecteur optique (67) qui détecte une intensité E du signal optique ; et
• un calculateur (65) qui calcule une valeur de compensation de signal (N) sur la base d'une intensité (E) de signal optique provenant de la source de lumière, et du signal photoacoustique (PA) et du signal photoacoustique A2 entrés par les moyens de détection, et calcule une concentration (C) du composant prédéterminé.

2. Appareil selon la revendication 1, dans lequel les moyens de détection comprennent des détecteurs de signaux.

3. Appareil selon la revendication 1, dans lequel les moyens de détection comprennent un premier moyen de détection qui détecte le signal photoacoustique PA et un second moyen de détection qui détecte le signal acoustique A2, et dans lequel le générateur de signal acoustique comprend un dispositif de génération/ mesure de signal acoustique qui comporte le second moyen de détection, et le détecteur optique comporte le premier moyen de détection.

4. Appareil selon l'une quelconque des revendications précédentes, dans lequel le dispositif de commande commande le générateur de signal acoustique de sorte que le signal acoustique A1 dans une bande de fréquence prédéterminée puisse être généré.

5. Appareil selon l'une quelconque des revendications précédentes, comprenant en outre un indicateur qui indique la concentration (C) du composant prédéterminé.

6. Appareil selon l'une quelconque des revendications précédentes, dans lequel la valeur de compensation (N) de signal satisfait à l'équation suivante :

$$N = \frac{PA}{E_S \sqrt{vA_2}}.$$

7. Appareil selon l'une quelconque des revendications précédentes, dans lequel la concentration (C) du composant prédéterminé est proportionnelle à la valeur de compensation (N) de signal.

8. Appareil selon l'une quelconque des revendications précédentes, dans lequel le détecteur optique, le dispositif de commande, et le calculateur sont intégrés dans une unité.

9. Appareil selon l'une quelconque des revendications précédentes, dans lequel le générateur de signal acoustique et les moyens de détecteur sont intégrés dans une unité.

10. Appareil selon l'une quelconque des revendications précédentes, dans lequel le dispositif de commande et le calculateur sont intégrés dans une unité.

11. Appareil selon la revendication 5, dans lequel le dispositif de commande, le calculateur et l'indicateur sont intégrés dans une unité.

12. Appareil selon la revendication 3, dans lequel le dispositif de génération/mesure de signal acoustique et le détecteur optique sont intégrés dans une unité.

13. Appareil selon l'une quelconque des revendications précédentes, dans lequel le générateur de signal acoustique peut être fixé à un corps humain au moyen d'un procédé de pompage d'air.

14. Appareil selon l'une quelconque des revendications précédentes, dans lequel la source de lumière est une quelconque parmi une diode laser (DL), une diode électroluminescente (DEL), un laser, un corps noir, et une lampe.

15. Procédé destiné à mesurer de manière non effractive des concentrations en fluide biologique comprenant les étapes consistant à :

• appliquer un signal optique ayant une bande de longueur d'onde prédéterminée qui peut être absorbée dans un composant prédéterminé d'un corps vivant à une partie prédéterminée du corps vivant ;

• détecter l'intensité (E) du signal optique et d'un signal photoacoustique (PA) généré lorsque des longueurs d'ondes prédéterminées du signal optique sont absorbées dans le composant prédéterminé du corps vivant ;

• générer un signal acoustique A1 ayant une bande de fréquence similaire à la bande de fréquence du signal photoacoustique (PA) à proximité de la partie prédéterminée du corps vivant ;

• détecter un signal acoustique A2 qui est un signal modulé du signal acoustique A1 en raison des caractéristiques acoustiques du corps vivant ; et

• calculer une valeur de compensation (N) de signal sur la base de l'intensité du signal optique, du signal photoacoustique PA et du signal acoustique A2, et calculer une concentration (C) du composant prédéterminé du corps vivant.

16. Procédé selon la revendication 15, dans lequel la valeur de compensation (N) de signal satisfait à l'équation suivante :

$$N = \frac{PA}{E_S\sqrt{vA_2}} \ .$$

17. Procédé selon la revendication 15 ou 16, dans lequel la concentration (C) du composant prédéterminé du corps vivant est proportionnelle à la valeur de compensation (N) de signal.

# FIG. 1 (PRIOR ART)

# FIG. 2 (PRIOR ART)

# FIG. 3

51 — LIGHT SOURCE

53

HUMAN BODY — 59

55

57

50

# FIG. 4

50

57'

53'

55'

# FIG. 5

# FIG. 6

```
┌─────────────────────────────┐
│      EMIT PHOTOACOUSTIC      │──── 101
│        SIGNAL (PA1)          │
└─────────────────────────────┘
              │
              ▼
┌─────────────────────────────┐
│     DETECT PHOTOACOUSTIC     │──── 103
│        SIGNAL (PA2)          │
└─────────────────────────────┘
              │
              ▼
┌─────────────────────────────┐
│      GENERATE ACOUSTIC       │──── 105
│        SIGNAL (A1)           │
└─────────────────────────────┘
              │
              ▼
┌─────────────────────────────┐
│       DETECT ACOUSTIC        │──── 107
│        SIGNAL (A2)           │
└─────────────────────────────┘
              │
              ▼
┌─────────────────────────────┐
│      CALCULATE SIGNAL        │
│   COMPENSATION VALUE(N)      │──── 109
│   & CONCENTRATION(C) OF      │
│     CERTAIN COMPONENT        │
└─────────────────────────────┘
```

# FIG. 7A

# FIG. 7B

# FIG. 7C

# FIG. 7D

WAVELENGTH (nm)

# FIG. 8